# EUROPEAN PATENT APPLICATION

(11) **EP 2 221 047 A1**
(43) Date of publication of application: **25.08.2010**
(21) Application number: 10164715.4
(22) Date of filing: 13.12.2002
(51) Int. Cl.: A61K 9/107, A61K 9/20, A61K 31/155, A61K 31/195, A61K 31/34, A61K 38/55

(54) **Reverse-micellar delivery system for controlled transportation and enhanced absorption of agents**

(30) Priority: 14.12.2001 US 24325
(62) Divisional of application: 02784963.7
(71) Applicant: MacGregor, Alexander, Markham, Ontario L6C 1A4 (CA)
(72) Inventor: MacGregor, Alexander, Markham, Ontario L6C 1A4 (CA)
(74) Representative: Main, Malcolm Charles

(57) **Abstract**

The invention can be summarized as follows. The present invention provides a reverse-micellar delivery system for enhanced absorption of an agent of interest across biological membranes such as the gastro-intestinal tract of mammals. The reverse-micelles comprise at least one ionic amphipathic compound, and at least one polar active agent ionizable in aqueous or physiological media. The delivery system facilitates transportation of the agent across the gastro-intestinal tract or other membranes and enhances the *in-vivo* release and availability of the agent(s) of interest within a fluid environment.

## Description

The present invention relates to a transmembrane transport delivery system for the controlled release of an agent of interest, as well as compositions and methods for preparing the delivery system. More particularly, the present invention provides a reverse-micellar transport system for dispensing an agent of interest to an environment of use.

### BACKGROUND OF THE INVENTION

Oral administration of drugs to the gastrointestinal tract (GIT) is a preferred method for both human and veterinary medicine. Most conventional drugs that are well absorbed from the intestines are transported across the GIT via transcellular or paracellular routes by a process of passive diffusion, although certain compounds are taken up by more specific mechanisms such as facilitated or active transport.

The therapeutic values and clinical significance of oral medications depends in part on the absorption of the active agent in the GIT. Impaired clinical advantages of several orally administered pharmaceutical drugs have been attributed to their poor absorption in the human GIT resulting in low bioavailability.

Non-polar molecules are generally readily absorbed due to their intrinsic lipophilicity and high partition coefficient in the mucosal cell membranes of the GIT.

Polar molecules with low partition coefficients such as metformin, cimetidine, ranitidine, sodium cromoglycate bisphosphonates (such as clodronate) and captopril often display poor or erratic absorption when dosed orally. The polypeptide and polysaccharide drugs such as insulin, calcitonin, parathyroid hormone or fractions or analogues thereof, luteinising hormone releasing hormone (LHRH) or analogues thereof (e.g. nafarelin, buserelin, goserelin), growth hormone, growth hormone releasing hormones, colony stimulating factors, erythropoietin, somatostatin, interferons and heparins cannot be given orally because, not only are they poorly absorbed due to their polar nature and size, but they can also be degraded by the endogenous enzymes present in the GIT. If such drugs are given orally, the absolute bioavailability (defined as the quantity reaching the systemic circulation) as compared to intravenous administration is generally low (typically less than 1% up to 60%).

Despite the inherent problems faced when attempting to administer polar drugs orally, various approaches have been proposed to improve oral absorption. Plausible strategies have included chemical modification to stabilise the drug and/or render it more lipid-soluble and hence improve its chances to diffuse across the lipid membrane or the GIT. Other researchers have added stabilising agents such as peptidase inhibitors (e.g. aprotinin) to reduce metabolic loss, while others have used various absorption promoting agents in the form of non-ionic surface active agents, bile salts and analogues thereof, phospholipids, chelating agents or acyl carnitine.

These previous attempts have been well documented and reviewed in the relevant literature. For example the various means to enhance the intestinal permeability of proteins, peptides and other polar drugs have been reviewed by Swenson and Curatolo (Advan. Drug Del. Rev. 8, 39, 1992). While mixed systems were reported these were restricted to systems comprising bile salt/oleic acid mixtures and polyethoxylated hydrogenated castor oil/oleic acid mixtures.

The ileocolonic delivery of insulin at 10 units/kg to a dog using a mixed micelle system comprising sodium glycocholate (30 mM) and a fatty acid (linoleic acid) of 40 mM has been described by Scott-Moncrieff and others, J. Pharm. Sci. 83, 1465 (1994). The reported bioavailability was 1.4%.

Medium chain glycerides (MCGs) have been reported to enhance the intestinal absorption of hydrophilic drugs. For example Beskid et al. (Pharmacology, 34, 77, 1988) reported that a formulation incorporating a mixture of glyceryl mono- and di-caprylate enhanced the absorption of an antibiotic from the intestinal tract of rats. Mixtures of medium chain glycerides with medium chain length fatty acids (C₈ - C₁₂) have also been reported (see for example Muranushi et al. Chem. Phys. Lipids 28, 269, 1981).

The use of anionic surfactants in solid pharmaceutical compositions is also known. Until recently, however, the presence of such surfactants was designed to facilitate fast and total release of the medicament from the composition (see, for example, Japanese Kokai 7320778 and A.A. Kassem et al, J. Drug Research, 1974, 6, 95).

U.S. Pat. No. 4,540,566 and P.B. Daly et al, Int. J. Pharm. 18, 201 (1984) describes a controlled release composition containing chlorpheniramine maleate, a cellulose ether and an anionic surfactant. However, the composition is a simple mixture and does not provide any particular advantages for drug delivery.

A delivery system that is capable of improving the absorption of orally administered polar drugs, especially those belonging to the Class III biopharmaceutics classification, which exhibit high solubility and poor permeability is desirable.

Further, there remains within the art a need for a reliable drug delivery system, that provides controlled drug delivery of highly ionised polar active agent(s) to an environment of use and that is amenable to physiological variables of the environment of use, such as the physiological pH or enzymes.

It is an obj ect of the present invention to overcome disadvantages of the prior art.

The above object is met by a combination of the features of the main claims. The sub claims disclose further advantageous embodiments of the invention.

### SUMMARY OF THE INVENTION

The present invention relates to a transmembrane transport delivery system for the controlled release of an agent of interest, as well as compositions and methods for preparing the delivery system. More particularly, the present invention provides a reverse-micellar transport system for dispensing an agent of interest to an environment of use.

According to the present invention there is provided a transmembrane delivery system comprising a reverse micelle and polar agent of interest. Preferably the reverse micelle comprises at least one amphipathic ionic compound, and the polar agent of interest comprises at least one polar ionizable agent. The amphipathic compound may be an anionic surfactant, cationionic surfactant or zwitterioinic surfactant capable of forming micelles in a fluid environment. Anionic surfactants may be selected from the group consisting of sodium or potassium dodecyl sulfate, sodium octadecylsulfate, sodium bis(2-ethylhexyl) sulfosuccinate (AOT), or a combination thereof. However other anionic surfactants may be employed. Similarly cationic surfactants may be selected from the group consisting of didodecyl dimethyl ammonium bromide (DDAB), cetyl-triammonium bromide (CTAB), cetylpyridinium bromide (CPB), dodecyl trimethyl ammonium chloride (DOTAC), sodium perfluorononanoate (SPFN), hexadecyl trimethyl ammonium bromide (HDTMA), or a combination thereof. However, other cationic surfactants may be employed in the delivery system of the present invention.

Also according to the present invention as defined above, there is provided a delivery system wherein the agent of interest is characterized by a partition coefficient between water and octanol at pH 7.4 of less than about 10.

The agent of interest may comprise, but is not limited to a therapeutic agent of interest, for example, but not limited to a therapeutically active compound of a Class III biopharmaceutics classification which exhibits high solubility and low permeability. For example, but not meaning to be limiting, the agent of interest may be selected from the group consisting of analgesic, anti-inflammatory, antimicrobial, amoebicidal, trichomonocidal agents, anti-Parkinson, anti-malarial, anticonvulsant, anti-depressants, antiarthritics, anti-fungal, antihypertensive, antipyretic, anti-parasite, antihistamine, alpha-adrenergic agonist, alpha blocker, anaesthetic, bronchial dilator, biocide, bactericide, bacteriostat, beta adrenergic blocker, calcium channel blocker, cardiovascular drug, contraceptive, decongestants, diuretic, depressant, diagnostic, electrolyte, hypnotic, hormone, hyperglycaemic, muscle relaxant, muscle contractant, ophthalmic, parasympathomimetic, psychic energizer, sedative, sympathomimetic, tranquilizer, urinary, vaginal, viricide, vitamin, non-steroidal anti-inflammatory, angiotensin converting enzyme inhibitors, polypeptide, proteins, sleep inducers or a combination thereof.

The delivery system as defined above may be formulated into a solid tablet, matrix tablet, granules or capsule. Further, the delivery system may comprise one or more pharmaceutically acceptable excipients, for example, but not limited to viscosity enhancers, enteric polymers, pH-specific barrier polymers, diluents, anti-adherents, glidants, binders, solubilizers, channeling agents, wetting agents, buffering agents, flavourants, adsorbents, sweetening agents, colorants, lubricants, or a combination thereof.

Further, according to the present invention as defined above, the delivery system may be formed by a matrix-type solid compact, by a compression or pelletization method, or a matrix-type extrusion spheroid, by a wet or dry extrusion method. Further, the delivery system may be granulated or microencapsulated to form particulates that may be compressed into solid compacts or filled into capsules. The dosage form may be selected from the group consisting of granulated, particulate, spheroidal, compact and dry blends. Optionally, the delivery system may be filled into capsules or suspended in a suitable liquid vehicle.

Also according to the present invention, there is provided the use of the delivery system to deliver one or more agents to a subject in need thereof. Preferably the agent is a therapeutic agent. The subject in need thereof may comprise any mammalian subject, for example, but not limited to a human subject.

Further according to the present invention, there is provided a method of delivering a therapeutic agent to a subject in need thereof. The method comprises,
i) formulating the delivery system with a therapeutic agent of interest and;
ii) administering the delivery system comprising the therapeutic agent of interest to a subject in need thereof.
The step of administering may comprise, but is not limited to oral administering.

This summary does not necessarily describe all necessary features of the invention but that the invention may also reside in a sub-combination of the described features.

### BRIEF DESCRIPTION OF THE DRAWINGS

These and other features of the invention will become more apparent from the following description in which reference is made to the appended drawings wherein:
**FIGURE 1** shows results of a comparative dissolution profile of glucophage XR (prior art) versus the delivery formulation of the present invention prepared according to Example 1A.
**FIGURE 2** shows results of a dissolution profile for the delivery system of the present invention prepared according to Example 1B.
**FIGURE 3** shows a diagrammatic representation of a two phase dissolution system that may be employed to measure transmembrane transport capability *in vitro.*
**FIGURE 4** shows a transport profile of the dye sunset yellow into the octanol layer of a water/octanol system, in the presence and absence of the cationic amphiphile cetylpyridinium chloride in the aqueous phase.
**FIGURE 5** shows a transport profile of the dye methylene blue into the octanol layer of a water/octanol system, in the presence and absence of the anionic amphiphile sodium lauryl sulphate in the aqueous phase.
**FIGURE 6** shows results of a comparative dissolution profile of Metformin release in a non-aqueous (Octanol) phase between Glucophage XR (Metformin) 500 mg extended release formulation (prior art) and the delivery system of the present invention comprising Metformin 500 mg Extended release formulation prepared according to Example 1A.
**FIGURE 7** shows comparative results of methformin profiles in healthy human subjects upon oral dosing with Glucophage XR (500mg metformin) and the delivery system of the present invention comprising metformin 500mg prepared according to Example 1A.
**FIGURE 8** shows results of comparative metformin release from glucophage XR (metformin 500mg) and the delivery system of the present invention comprising Metformin 500 mg Extended release formulation prepared according to Example 1B:

### DESCRIPTION OF PREFERRED EMBODIMENT

The invention relates to a transmembrane transport delivery system for the controlled release of an agent of interest, as well as compositions and methods for preparing the delivery system. More particularly, the present invention provides a reverse-micellar transport system for dispensing an agent of interest to an environment of use.

The following description is of a preferred embodiment by way of example only and without limitation to the combination of features necessary for carrying the invention into effect.

According to an aspect of an embodiment of the present invention, there is provided a transmembrane delivery system comprising a reverse micelle and a polar agent of interest. Preferably, the reverse micelle comprises at least one amphipathic ionic compound, and the polar agent of interest comprises at least one polar ionizable agent of interest, for example but not limited to therapeutic active agents.

By the term "amphipathic ionic compound" or "amphiphilic ionic compound" it is meant any compound, synthetic or otherwise, whose molecules or ions have a certain affinity for both polar and non-polar solvents. As used herein, the term "amphipathic compounds" is meant to be synonymous with the term "amphiphilic compounds".

Depending on the number and nature of the polar and non-polar groups present in an amphipathic ionic compound, the compound or amphiphile may be predominantly hydrophilic (water loving), lipophilic (oil loving), or reasonably balanced between these two extremes. For example, but not wishing to be limiting, ionic surfactants are a class of amphiphilic ionic compounds.

Surfactants can be classified by reference to the nature of the hydrophilic region, which can be anionic, cationic, zwitterionic or non-ionic. In the present invention, ionic surfactants or mixtures thereof are preferred.

Anionic, cationic or zwitterionic surfactants may be employed in the reverse micellar delivery system of the present invention. Examples of anionic surfactants which may be employed by the present invention include, but are not limited to surfactants which exhibit favourable packing geometry of the surfactant molecule in the interfacial area, such as, but not limited to sodium dodecyl sulphate (SDS) and sodium bis (2-ethylhexyl) sulfosuccinate (AOT). Other anionic surfactants which may be employed in the delivery system include, but are not limited to alkali metal sulphates, such as sodium or potassium dodecyl sulphate, sodium octadecylsulphate, alkali metal sulphonates, such as alkali metal salts of benzene sulphonates, naphthalene sulphonates and, dialkysulphosuccinates. In an aspect of an embodiment of the present invention, the anionic surfactant is an alkali metal sulphonate, for example, but not limited to an alkali metal salt of benzene sulphonate, naphthalene sulphonate and dialkysulphosuccinate.

Cationic surfactants which may be employed by the present invention include, but are not limited to didodecyl dimethyl ammonium bromide (DDAB), cetyl-triammonium bromide (CTAB), cetylpyridinium bromide (CPB), didodecyl dimethyl ammonium bromide, (DDAB), dodecyl trimethyl ammonium chloride (DOTAC), sodium perfluorononanoate (SPFN), and hexadecyl trimethyl ammonium bromide. However, any cationic surfactant which is capable of forming reverse micelles may be employed in the delivery system of the present invention.

As would be evident to someone of skill in the art, it is generally preferred that the surfactant or surfactants employed in the delivery system of the present invention be cleared for human ingestion. Therefore, surfactants with' a low toxicity are preferred. For example, but not wishing to be limiting in any manner, surfactants having an LD50 exceeding about 10 g/kg are preferred. More preferably the surfactants exhibit an LD50 exceeding about 15 g/kg. The absence of other side effects is also desirable. Although surfactants which have already been approved for human ingestion are preferred, other surfactants may be employed in the delivery system of the present invention.

The "critical micelle concentration" (CMC) defines the minimum amount of surfactant required to form micelle-phase in a particular solvent, and may be considered to represent the solubility of the surfactant monomer in that solvent. The "critical reverse micelle concentration" (CrMC) as used herein defines the minimum amount of surfactant required to form the reverse micelle phase in a particular solvent containing specific ions.

At surfactant concentrations well above the CMC any small amounts of monomeric surfactant (and perhaps small pre-micellar surfactant aggregates) exists in equilibrium with the bulk of the surfactant in micellar aggregates.

The solubility of surfactant monomer in a particular solvent is dependent on specific solvent-solute forces. Without wishing to be bound by theory, the dominant intermolecular interactions between polar surfactant, and alkane solvent, molecules are thought to be dipole-induced dipole, and the induced dipole-induced dipole, forces.

By the term "ionic monomer" it is meant cationic and anionic monomers, i.e. monomers wherein the part of the monomer molecule containing an ethylenically unsaturated group has a positive or negative charge, respectively.

The capacity of ionic monomers to form inverted micelles can be determined by standard tests known in the art for determining critical micelle concentration (CMC). As is known to one skilled in the art, some of the properties of a surfactant solution, such as refractive index, light scattering, interfacial tension, viscosity, dye solubilization and absorption of fluorescent substance usually vary linearly with increasing concentration up to the CMC, at which point there is a break or change in one or more of these properties (Encyclopaedia of Chemical Technology, Kirk-Othmer - 3rd. ed. Vol. 22, A Wiley Interscience Publication - New York (1983) Page 354; which is incorporated herein by reference).

### Formation of Reverse Micelles

Reverse micelles have a polar core, with solvent properties dependent upon the [water]/[surfactant] ratio (W), which can solvate highly polar water soluble compounds (e.g. hydrophilic substances such as proteins, enzymes, ionised drugs, chemical catalysts and initiators) and sometimes even normally insoluble amphiphilic compounds. At low W values, the water in the micelle is highly structured due to its association with the ionic groups on the surfactant molecule and the counter ion core. The environment in the micelle core resembles that of an ionic fluid due to the large counter ion concentration. At larger W values, the swollen micelles (or microemulsions) are thought to have a free water core which provides a distinct third solvent environment and which approaches the properties of bulk water. Certain enzymes and polar compounds are only solubilized by reverse micelles swollen by large amounts of water, (W greater than about 10).

As described in more detail below, and without wishing to be bound by theory, when ionic amphiphiles are introduced into a hydrophilic fluid, and provided that the concentration of the amphiphile is at or above their intrinsic CMC values, aggregation occurs with the formation of micelles. The aggregate composition in the micelles is oriented such that the hydrocarbon chains face inward into the micelle to form their own lipophilic environment, while the polar regions surrounding the hydrocarbon core are associated with the polar molecules in the hydrophilic fluid continuous phase. The orientation of micellar aggregates in non-polar fluid environment is essentially reversed. The polar regions face inwards into the micelles while the hydrocarbon chains surrounding the core of the micelles interact with the non-polar molecules in the fluid environment.

When present in a liquid medium at low concentrations, the amphiphiles exist separately and are of such a size as to be sub-colloidal. As the concentration is increased, aggregation occurs over a narrow concentration range. These aggregates which are composed of several monomers are called micelles. The concentration of monomers at which micelles are formed is termed the Critical Micelle Concentration, or CMC.

It is well known in the art that ionic amphiphiles, such as anionic or cationic surfactants, produce micelles in hydrophilic solvents by forming a lipophilic core through aggregation of the hydrocarbon chain. Polar heads of these compounds surrounding the core of the micelles interact and associate with the polar molecules in the fluid environment. As described herein, it has been unexpectedly observed that reverse micelles with polar cores can exist in hydrophilic fluids, and that such reverse micelles and microemulsions have unique, useful properties that can provide for transportation and delivery of polar ionizable compounds across biological membranes.

When ionic amphiphiles are introduced into a hydrophilic fluid media composed of polar molecules whose ionization characteristics results in molecular or ionic charges opposite to that of the amphiphilic polar heads, an association colloid may be formed with a reverse orientation to that which is ordinarily expected. The charged polar region of the amphiphile associates with the oppositely charged polar molecules or ions of the fluid environment. At a certain concentration of the amphiphile, association colloids may be formed. These colloids comprise reverse-micelles with a polar core comprised of the oppositely charged ions or molecules in fluid media in association with the polar heads of the amphiphile.

Such reverse-micelles are surrounded by the lipophilic regions of amphiphile in a colloidal internal phase and separated from the hydrophilic fluid continuous phase.

Hydrophilic drugs that are highly ionizable in a prevailing physiological environment such as the gastro-intestinal lumen are thought to be poorly absorbed in part due to their polarity and charges. While these groups of compounds are soluble in the aqueous physiological media of the GIT, they exhibit poor partition coefficients and low permeabilities across the membranes of the GIT. Several therapeutic agents belonging to these categories of compounds, sometimes referred in the art as Class III (high solubility, low permeability) biopharmaceutical compounds often show saturable absorption kinetics together with low bioavailabilities. The reverse-micelle delivery system of the present invention enhances GIT transmembrane transport and delivery of these compounds.

Once dissolved in the physiological fluid environment, polar agents exist primarily as charged ions or molecules. Reverse-micelles formed in these conditions are composed of bound agents in the core of the micelles, surrounded by lipophilic hydrocarbons. The bound ionised agents are thought to be encapsulated in spherical colloidal reverse-micelles. These reverse micelle colloids partition across the lipophilic mucosal membranes of the GIT- thus acting as transport carriers for the therapeutic agents. Once partitioned across the lipophilic membranes, the reverse micelles disassociate as the concentration within the membrane falls below the CMC or CrMC and the interfacial tension drops in the lipophilic environment.

### Reverse-Micellar Delivery System

When the reverse-micellar delivery system of the present invention comes into contact with an external fluid of the environment, such as water or other biological fluid, a burst or gradual release of the ionic amphiphiles may occur. A concurrent release of the additional ionic amphiphiles and the agent of interest follows.

The ionic amphiphiles released dissolve in the aqueous fluid media forming ionic monomers. Upon release of agent(s) of interest, depending on the prevailing pH of the fluid environment and the pKa of the chemical compound, ionised molecules are formed. These ions carry permanent charges opposite to that of the polar region of the ionic amphiphiles. The oppositely charged polar groups of the ionised agents of interest and amphiphiles attract each other. Without wishing to be bound by theory, at some point when sufficient ionic monomers of the amphiphile are attracted to the charged species in the aqueous fluid, aggregation and reverse micelle formation occurs. This point is believed to be the critical reverse micelle concentration (CrMC). These reverse micelles, in the aqueous fluid environment, eventually form colloidal microemulsions. In the human GIT, such reverse micelles are in direct contact with the lipophilic membranes of the absorbing mucosal cells. Due to the inherent lipophilicity of the outer surface of the reverse-micelles, they partition rapidly into these membranes, thereby facilitating absorption.

Without wishing to be bound by theory, once the reverse micelles partition into the lipophilic membrane, the concentration of the amphiphilic molecule component of the reverse micelles diminish beneath the CMC or CrMC. The reverse micelles undergo disaggregation and release the polar agent within their core. The kinetics of transport and transmembrane release of these agents may be essentially zero order or near about zero order.

According to the present invention, the term "polar agent" is used to include compounds with a partition coefficient between water and octanol at pH 7.4 of less than about 10. Preferably the polar agent is soluble in physiological fluid and is highly ionizable at the prevailing pH. It is contemplated that one or more polar agents or mixtures of polar agents maybe combined for administration as described herein.

The polar agent may be a therapeutic agent such as a polar drug. In such an embodiment the drug preferably has a molecular weight from about 100 Da to about 100000 Da. Further, the polar drug is preferably an active drug but it may be a drug in a masked form such as a prodrug. The term "active drug" is meant to include compounds which are therapeutically, pharmacologically, pharmaceutically, prophylactically or diagnostically active, that produce a localized or systemic effect or effects in animals, for example, but not limited to mammals, humans and primates.

Therapeutic agents, pharmacologically active agents, or other preferably polar agents also include, but are not limited analgesics, anti-inflammatories, anti-microbials, amoebicidals, trichomonocidal agents, anti-Parkinson, anti-malarial, anti-convulsant, anti-depressants, antiarthritics, anti-fungal, anti-hypertensive, anti-pyretic, anti-parasite, antihistamine, alpha-adrenergic agonist, alpha blocker, anaesthetic, bronchial dilator, biocide, bactericide, bacteriostat, beta adrenergic blocker, calcium channel blocker, cardiovascular drug, contraceptive, decongestants, diuretic, depressant, diagnostic, electrolyte, hypnotic, hormone, hyperglycaemic, muscle relaxant, muscle contractant, ophthalmic, parasympathomimetic, psychic energizer, sedative, sympathomimetic, tranquilizer, urinary, vaginal, viricide, vitamin, non-steroidal anti-inflammatory, angiotensin converting enzyme inhibitors, polypeptide, proteins, sleep inducers, or a combination thereof, as would be evident to one of skill in the art.

Drugs which may be employed as polar agents of interest in the delivery system of the present invention include, but are not limited to metformin, cimetidine, ranitidine, sodium cromoglycate, gabapentin and bisphosphonates such as clodronate and captopril, polypeptide drugs such as, but not limited to insulin, calcitonins, parathyroid hormone, luteinising hormone releasing hormones such as, but not limited to nafarelin, buserelin, and goserelin, growth hormone, growth hormone releasing hormones, colony stimulating factors, erythropoietin, somatostatin and analogues such as, but not limited to octreotide and vapreotide, α-interferon, β-interferon, γ-interferon, proinsulin, glucagon, vasopressin, desmopressin, thyroid stimulating hormone, atrial peptides, tissue plasminogen activator, factor VIII, cholecystokinin, octreotide, polysaccharide drugs such as, but not limited to low molecular weight heparin, genes such as DNA or DNA constructs and antisense agents, or a combination thereof, as would be evident to one of skill in the art. Further the present invention also contemplates variants, analogues and derivatives of these and other drugs as polar agents of interest in the delivery system of the present invention.

Examples of other polar agents of interest are disclosed in Remington's Pharmaceutical Sciences (16th Ed., 1980, published by Mack Publishing Co., Easton, Pa.; and in The Pharmacological Basis of Therapeutics, by Goodman and Gilman, 6th Ed., 1980, published by The MacMillian Company, London, which is herein incorporated by reference). Furthermore, an agent of interest may include, but is not limited to, pesticides, herbicides, germicides, biocides, fungicides, algicides, insecticides, rodenticides, antioxidants, preservatives, plant growth inhibitors, plant growth promoters, chemical reactants, disinfectants, sterilization agents, foods, fermentation agents, food supplements, cosmetics, nutrients, vitamins, pharmaceutical drugs, nutraceuticals, vitamins, sex sterilants, fertility promoters, fertility inhibitors, micro-organism attenuators, air purifiers, or other agents that benefit the environment of their use.

Other agents of interest include, but are not limited to, organic and inorganic compounds in various forms, such as charged molecules, molecular complexes, pharmacologically acceptable salts such as hydrochlorides, hydrobromides, palmitate, phosphate, sulphate laurylate, nitrate, borate, maleate, tartrate, acetate, salicylate and oleate. Prodrugs and derivatives of drugs such as esters, ethers and amides are also included.

One or more agents of interest, preferably a polar agent, can be in the delivery system of the present invention in form of solid particles, granules, microencapsulated solid, microencapsulated liquid, powder and coated particles, for example, the agent of interest may comprise a plurality of discrete active particulates. Water insoluble agents of interest can be used in a form that renders it water soluble, and upon release from the delivery system, they may be converted to their original, or biologically active form, by enzyme hydrolysis, by pH, or metabolic processes, depending on the environment of use.

The delivery system may also comprise an entric coating, or one or more pH sensitive barrier polymers. The delivery system may be
i) a matrix-type solid compact, for example, made by a compression or pelletization, a matrix-type extrusion spheroid, made by a wet or dry extrusion;
ii) granulated or microencapsulated to form particulates that may be compressed into solid compacts or filled into capsules; or
iii) spheroidal, compact, comprising dry blends, filled into capsules or suspended in a suitable liquid vehicle.
Furthermore, the delivery system as described herein may be combined with suitable agents that effect the rate and duration of delivery release as required. For example which is not to be considered limiting in any manner, hydroxyl propyl methyl cellulose phthalate (HPMCP 55) may be added for delayed release (see Example 1B). Other additions may be added as would be known to one of skill in the art.

The delivery system may also be dispersed prior to administration to a subject so that the reverse micelles are formed in the dispersed mixture. For example, which is not to be considered limiting in any manner, the delivery system of the present invention may be dispersed within a liquid, and the liquid administered in an oral, or injectable form as required.

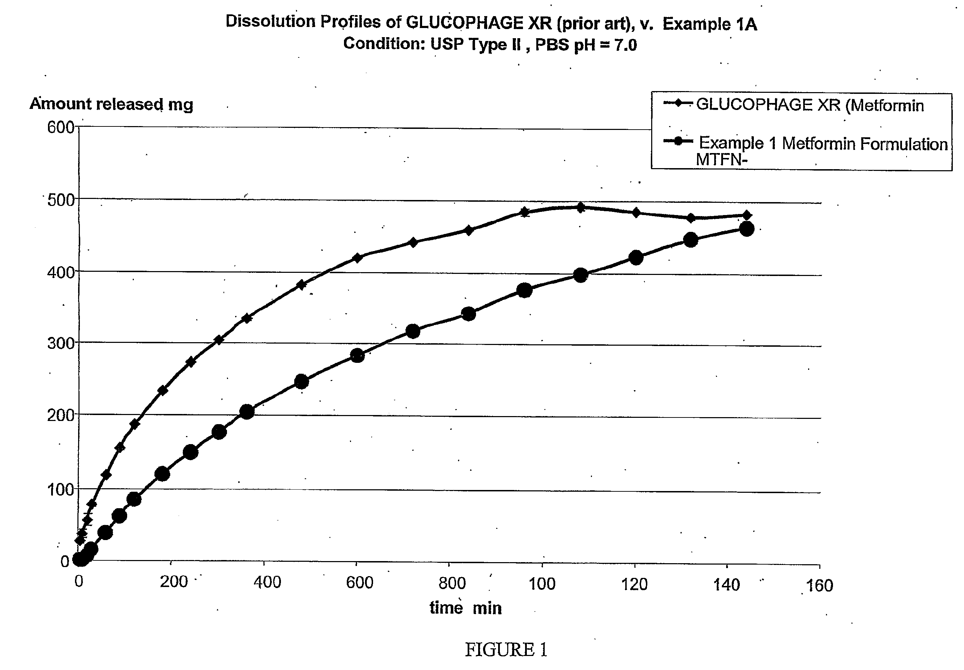

Referring now to Figure 1, there is shown a comparative dissolution profile of Glucophage XR (500 mg metformin) with the reverse micelle delivery system comprising 500 mg metformin prepared as described in Example 1A (extended release formulation). The results demonstrate that the reverse micelle delivery system maybe employed to deliver an agent of interest, for example, but not limited to a therapeutic agent of interest.

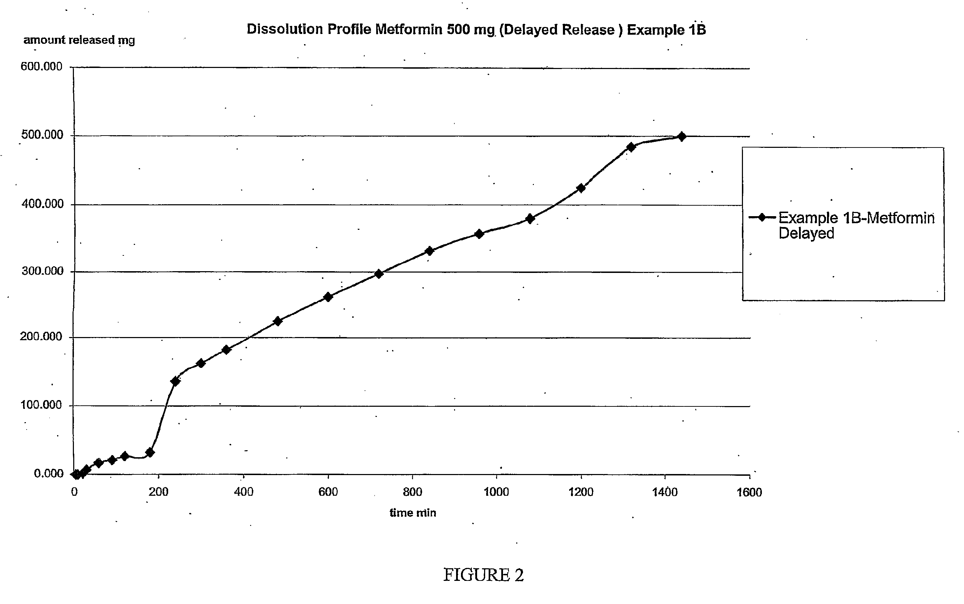

Referring now to Figure 2, there is shown a dissolution profile of 500 mg metformin formulated in the reverse micelle delivery system of the present invention prepared according to Example 1B (delayed release formulation). The results suggest that the delivery system of the present invention may be employed to deliver polar drugs such as metformin and other polar agents.

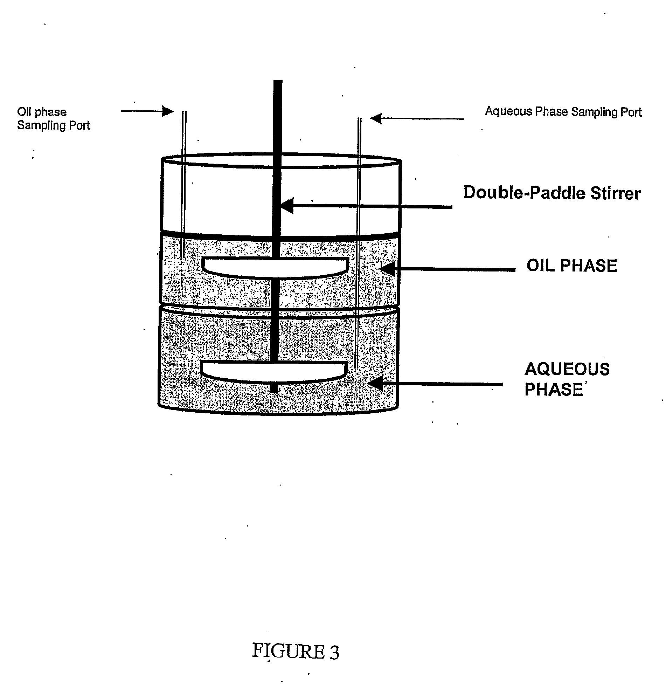

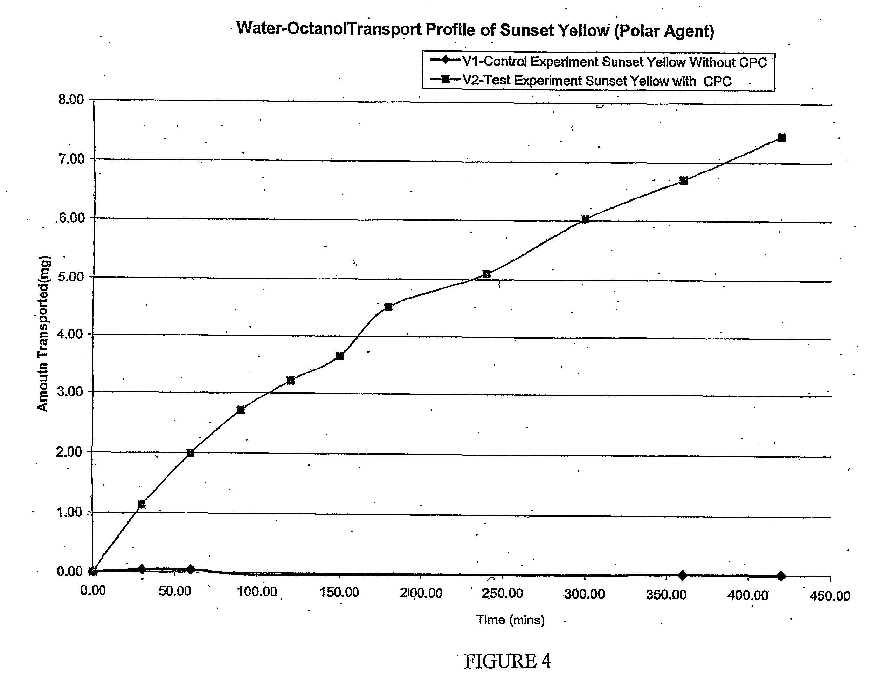

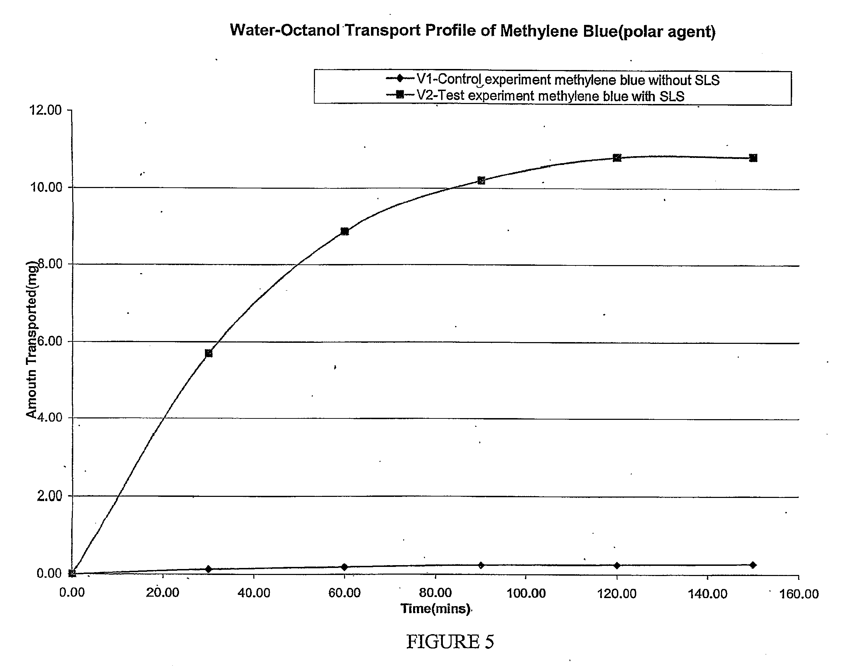

Referring now to Figure 3, there is shown a diagrammatic representation of a two-phase dissolution system that may be employed to measure transmembrane transport capability of delivery systems *in vitro.* With reference to Figure 4, there is shown a transport profile of the dye sunset yellow into the octanol layer of a water/octanol system, in the presence and absence of the cationic amphiphile cetylpyridinium chloride in the aqueous phase. Referring to Figure 5, there is shown a transport profile of the dye methylene blue into an octanol layer of a water/octanol system, in the presence and absence of the anionic amphiphile sodium lauryl sulphate in the aqueous phase. The results illustrated in Figures 4 and 5 indicate that in the absence of an amphiphilic compound in the aqueous phase, no significant transport of the polar dye into the octanol layer can take place. When both the polar dye and an amphiphilic compound are initially added to the aqueous phase, however, reverse micelles are formed, which can cross over the octanol/water interface into the octanol layer.

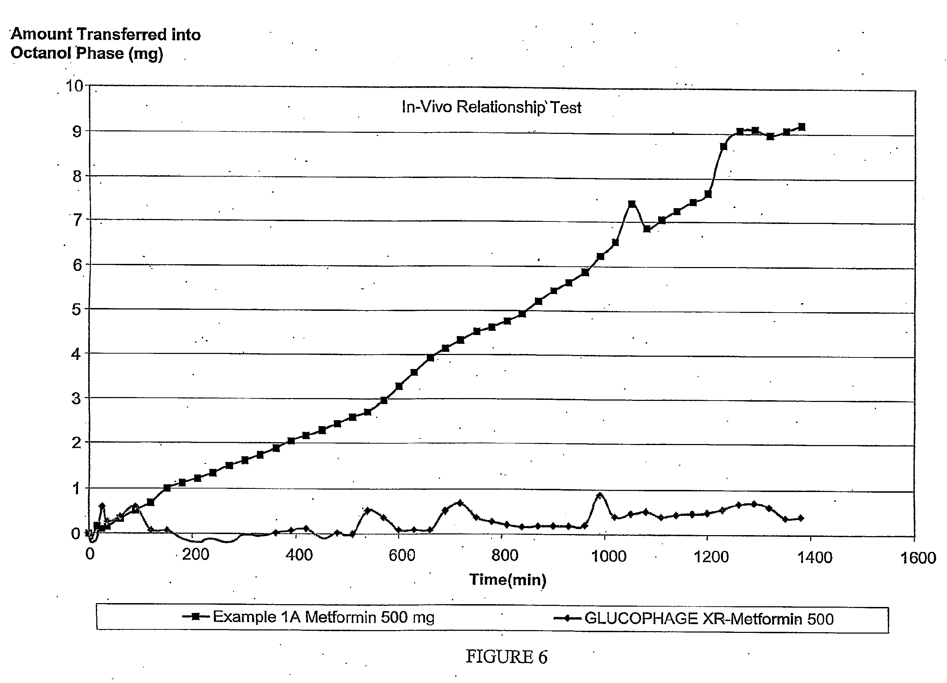

Shown in Figure 6 is a comparative profile showing metformin release into a non-aqueous octanol phase for the Glucophage XR (500mg metformin) delivery system known in the art, and the reverse micelle delivery system of the present invention (prepared according to Example 1A, an extended release formulation).

In general, the results shown in Figures 4-6 demonstrate that the reverse micelle delivery system enhances transfer of a polar agent, for example, but not limited to a polar therapeutic agent into a relatively non-polar environment. These results further suggest that the reverse micelle delivery system of the present invention may enhance delivery of a polar agent through the GIT and into the systemic circulation of a subject.

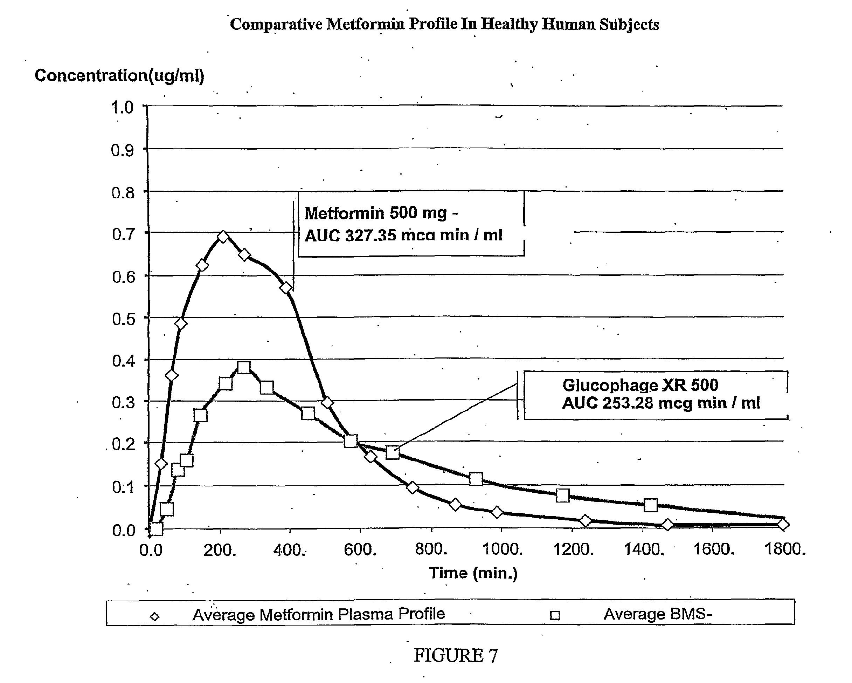

With reference to Figure 7, there is shown a comparative metformin plasma profiles for Glucophage XR (500mg metformin) delivery system known in the art and the reverse micelle delivery system of the present invention which contains the equivalent amount of metformin (500mg; extended release formulation, prepared according to Example 1A). The results shown in Figure 7 indicate that the metformin formulation of the present invention exhibits an AUC of about 330 mcg min/ml whereas the Glucophage XR 500 formulation exhibits an AUC of about 250 mcg min/ml, suggesting that the reverse micellar delivery system of the present invention exhibits greater bioavailability compared to other formulations known in the art.

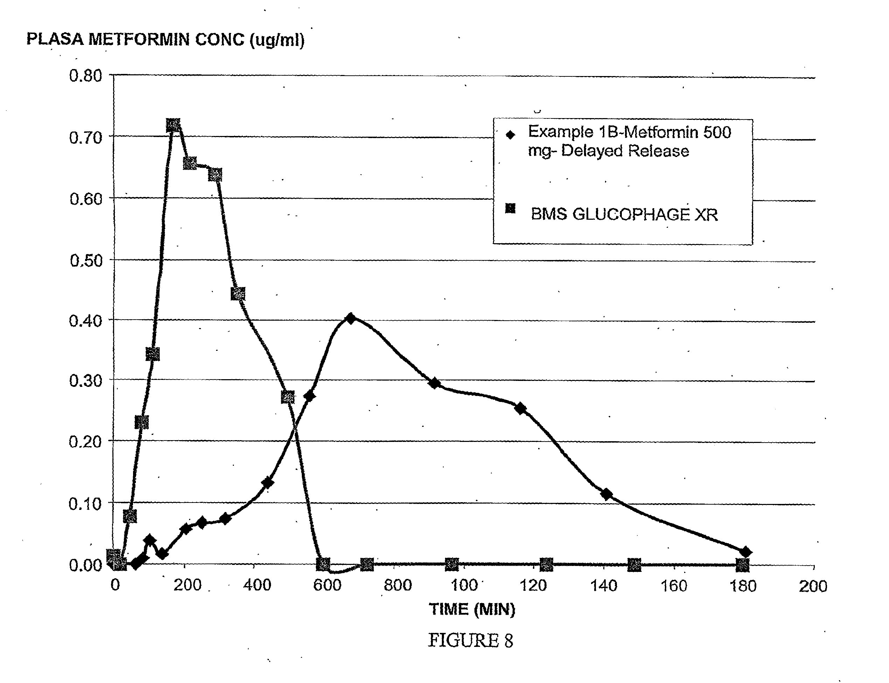

Referring now to Figure 8, there is shown comparative metformin plasma profiles for the Glucophage XR (500mg metformin) delivery system known in the art and the reverse micelle delivery system of the present invention, prepared according to Example 1B (delayed release formulation). The results indicate that the reverse micelle delivery system of the present invention is capable of delivering a more uniform dose over a longer time period than other formulations known in the art. Thus, the reverse micelle delivery system of the present invention may improve the bioavailability and enhance the uniformity of the bioavailable dose when administered to a subject.

In an alternate aspect of a embodiment of the present invention, there is provided a reverse micelle delivery system comprising at least one ionic amphipathic compound or surfactant in a matrix composition, the matrix composition containing one or more agents of interest with or without other pharmaceutical adjuvant(s). The delivery system of the present invention permits the release of one or more agents of interest in a controlled manner, with a first-order, zero-order or near zero-order release kinetics, over a therapeutically practical time period. Examples of extended release, or delayed release formulation are presented in Example 1.

In a further aspect of a embodiment of the present invention there is provided a solid pharmaceutical dosage form, for example, but not limited to matrix based solid compact suitable for oral administration wherein the delayed release is brought about by use of suitable excipients that are industrially available, non-toxic and easy to process. The pharmaceutical dosage form includes, for example, but not limited to, compressed tablets, granules, pellets, suspensions, extrusion spheroids or compacts obtained by direct compression, wet granulation, dry granulation, hot melt granulation, microencapsulation, spray drying, and extrusion methods as would be evident to one of skill in the art. Other solid dosage forms such as hard gelatine capsules can also be.derived from dry blends, granulations, suspensions, spheroids, pellets, tablets and combinations therefrom, as are commonly known in the art.

The pharmaceutical dosage form may also include excipients as required, for example, but not limited to one or more viscosity enhancers, enteric polymers, pH-specific barrier polymers, diluents, anti-adherents, glidants, binders, plasticizer, solubilizers, channelling agents, stabilizers, compaction enhancers, wetting agents, fillers, buffering agents, flavourants, adsorbents, sweetening agents, colorants, lubricants, or a combination thereof.

Formulations incorporating solid dosage forms may further include one or more additional adjuvants, which can be chosen from those known in the art including flavours, colours, diluents, binders, plasticizers, fillers, surfactant, solubilizers, stabilizers, compaction enhancers, channelling agents, glidants, lubricants, coating polymers and anti-adherents.

The dosage forms and reverse micelle delivery system as taught herein may be used in pharmaceutical, veterinary, food, pesticidal, horticultural, herbicidal, agricultural, cosmetic, industrial, cleansing, and confectionery applications.

Also according to the present invention, there is provided the use of the delivery system to deliver one or more agents to a subject in need thereof Preferably the agent is a drug or a therapeutic agent. The subj ect in need thereof may comprise any mammalian subject, for example, but not limited to a human subject.

Further according to the present invention, there is provided a method of delivering a therapeutic agent to a subject in need thereof. The method comprises,
i) formulating the delivery system with a therapeutic polar agent of interest and;
ii) administering the delivery system.comprising the therapeutic agent of interest to a subject in need thereof.

The step of administering may comprise, but is not limited to oral administering.

The above description is not intended to limit the claimed invention in any manner, Furthermore, the discussed combination of features might not be absolutely necessary for the inventive solution.

The present invention will be further illustrated in the following examples. However, it is to be understood that these examples are for illustrative purposed only, and should not be used to limit the scope of the present invention in any manner.

### Examples

### Example 1: Preparation of Reverse-Micellar Matrix Tablets

The reverse micellar delivery system can be prepared by simple matrix tablet manufacturing process. The agent of interest is first screened to obtain a particle size distribution suited for the ionic amphiphile. The screened agent is mixed thoroughly in a high shear mixer for about 2 - 5 minutes. The blend achieved is tested for homogeneity. The resulting mixture is further mixed with other suitable excipients required to form a polymeric matrix composition. Optionally the polymeric composition may be achieved by a number of conventional granulation techniques such wet, dry, hot melt or extrusion granulation. Whatever the method, the matrix composition may be further lubricated and compressed on a suitable tablet press to form a compact. Such compact may be further coated with a polymeric composition comprised of a desired polymer and the ionic amphiphile. The coating techniques are known within the art.

A manufacturing process for a reverse micellar delivery system in the form of a matrix tablet generally involves, but is not limited to the following steps:
a) Preparation of ionic amphiphile - polar agent mixture;
b) Preparation of polymeric matrix components;
c) Blending or granulating a) and b) to form reverse micellar mixture or granules;
d) Compressing the blend into a suitable compact;
e) Preparation of coating polymer-ionic amphiphile composition;
f) Coating the compact with the coating preparation e).

### Example 1A. Metformin Hydrochloride 500 mg Extended Release Tablet

| **Component** | **% Per Unit Tablet** |
|---|---|
| Metformin Hydrochloride USP | 69 |
| Cetyl Alcohol NF | 18 |
| Sodium Lauryl Sulphate NF | 10 |
| Ethyl Cellulose NF | 2 |
| Magnesium Stearate | 1 |

### Example 1B. Metformin Hydrochloride 500 mg Delayed Release Table

| **Component** | **% Per Unit Tablet** |
|---|---|
| Metformin Hydrochloride USP | 65 |
| Cetyl Alcohol NF | 18 |
| Sodium Lauryl Sulphate NF | 13 |
| Hydroxyl Propyl Methyl Cellulose Phthalate HPMCP 55 | 2 |
| Ethyl Cellulose NF | 1 |
| Magnesium Stearate | 1 |

### Example 1C. Ranitidine Hydrochloride 300 mg Extended Release Tablet

| **Component** | **% Per Unit Tablet** |
|---|---|
| Metformin Hydrochloride USP | 70 |
| Cetyl Alcohol NF | 18 |
| Sodium Lauryl Sulphate NF | 9 |
| Ethyl Cellulose NF | 2 |
| Magnesium Stearate | 1 |

### Example 1D. Gabapentin Hydrochloride 400 mg Extended Release Tablet

| **Component** | **% Per Unit Tablet** |
|---|---|
| Gabapentin Hydrochloride USP | 62 |
| Cetyl Alcohol NF | 18 |
| Sodium Lauryl Sulphate NF | 18 |
| Ethyl Cellulose NF | 1 |
| Magnesium Stearate | 1 |

### Example 1E. Captopril Hydrochloride 100 mg Controlled Delivery Tablet

| **Component** | **% Per Unit Tablet** |
|---|---|
| Metformin hydrochloride USP | 63 |
| Cetyl alcohol NF | 18 |
| Ascorbic Acid NF | 8 |
| Cetyl Triammonium bromide NF | 9 |
| Ethyl Cellulose NF | 1 |
| Magnesium Stearate | 1 |

### EXAMPLE 2: Measurement of Drug Release in Vitro

The conventional USP dissolution testing can assess the *in-vitro* drug release from the delivery system. The following testing conditions are used:

| | |
|---|---|
| Test Media: | Phosphate Buffer pH 6.8 or De-ionised Water pH 7 |
| Volume: | 900 ml or 1000 ml |
| Temperature: | 37 degree Celsius +/- 0.5 degrees |
| Agitation Speed: | 40 rpm, 50 rpm, or 100 rpm |
| Apparatus type: | Type II (Paddle) or Type I (Basket) |

The quantity of active component released is measured from aliquots of samples taken over a duration of 6, 12, or 24 hours. The compound may be quantified by UV Spectrophotometry or by HPLC analysis.

A comparative dissolution profile of a polar aget, for example, metformin hydrochloride 500 mg extended release tablets prepared as described above (Example 1A) and a prior art extended release formulation (Glucophage XR) is shown in Figure 1. The tablets were tested in a type II dissolution apparatus in PBS pH 7.0.

A dissolution profile of metformin 500 mg delayed release tablets prepared as described above (Example 1B)is shown in Figure 2. The tablets were tested in a type II dissolution apparatus using simulated gastric fluid media (SGF) pH 2.5 initially for 3 hours followed by simulated intestinal fluid media (SIF) pH 6.8 for a further 21 hours.

### EXAMPLE 3: In-vitro Experimental Evidence of Reverse-Micellar Transport

The novel reverse micelles according to the present invention are formed in a single phase aqueous fluid environment. These reverse micelles are transportable across a lipophilic biological membrane. To demonstrate the existence and transportability of reverse micelles according to the present invention, experiments were conducted using a two-phase water-octanol system as shown in Figure 3 to monitor the transportation of reverse micelles formed in the water phase into the organic phase. The water/octanol system is used to mimic the aqueous/lipophilic biological membrane interface through which the reverse micelles of the present invention are transported *in vivo.*

### Transport of reverse micelles comprising a polar acidic dye and a cationic surfactant.

In this experiment, a polar acidic dye, and a cationic surfactant (amphiphilic compound) were initially added to an aqueous layer to form a colored solution. Thereafter, a layer of octanol was slowly added to form a clear distinct layer above the aqueous colored solution. Both liquid layers were stirred at 50 rpm. Samples of 4ml were removed from the top (octanol) layer and analyzed spectrophotometrically at 480 nm for dye content. The sampling times were 30min, 60min, 90 min, 120min, 150min, 180 min, 240 min, 300 min, 360 min, and 420 min post dissolution of the compounds in the aqueous layer. The migration of the dye into the octanol phase was also tracked by visually monitoring the color change of the octanol layer over time. A control experiment was simultaneously run, in which no cationic surfactant (amphiphile) was present. The results of the experiments are shown in Figure 4. The specific details relating to the set-up of the experiment are shown below:

| | |
|---|---|
| Cationic amphiphile: | Cetylpyridinium chloride (CPC) USP |
| | Amount used: 20.52 mg or 0 mg (control) |
| Polar acidic dye: | Di-sodium 2-hydroxy-1-(4-sulfonatophenylazo) naphthalene-6-sulfonate (sunset yellow or FD&C yellow # 6; absorbance maxima in de-ionized water pH 7 of 485 nm) Amount used: 30.32 mg |
| Test system: | Modified USP type II dissolution apparatus comprised of two-layer water-octanol dissolution media plus a double paddle stirrer. |
| Aqueous media: | 600 ml De-ionized water (pH 7) |
| Lipophilic media: | 400 ml Octanol |
| Temperature: | 37°C |
| Rotation Speed: | 50 rpm |

The experiment in which the polar dye and the amphiphilic compound were both initially added to the aqueous layer resulted with time in the progressive development of yellow color, and in an increase in the concentration of the polar agent in the octanol layer. In contrast, in the control experiment, there was no significant color development or increase in the concentration of the polar agent in the octanol layer. These results therefore indicate that in the absence of an amphiphilic compound, the polar agent is not significantly transported into the octanol layer. In the presence of an amphiphilic compound, however, reverse micelles can be formed with the polar agent, which can cross the water/octanol interface into the octanol phase.

### Transport of reverse micelles comprising a polar basic dye and an anionic surfactant.

In this experiment, a polar basic dye, and an anionic surfactant (amphiphilic compound) were initially added to an aqueous layer to form a colored solution. Thereafter, a layer of octanol was slowly added to form a clear distinct layer above the aqueous colored solution. Both liquid layers were stirred at 50 rpm. Samples of 4ml were removed from the top (octanol) layer and analyzed spectrophotometrically at 480 nm for dye content. The sampling times were 30min, 60min, 90 min, 120min, 150min, 180 min, 240 min; 300 min, 360 min, and 420 min post dissolution of the compounds in the aqueous layer. The migration of the dye into the octanol phase was also tracked by visually monitoring the color change of the octanol layer over time. A control experiment was simultaneously run, in which no anionic surfactant (amphiphile) was present. The results of the experiments are shown in Figure 5. The specific details relating to the set-up of the experiment are shown below:

| | |
|---|---|
| Anionic amphiphile: | Sodium lauryl sulphate USP Amount used: 20.62 mg or 0 mg (control) |
| Polar basic agent: | 3, 7-bis (dimethylamino) phenothiazin-5-ium chloride, (basic blue 9, methylene blue USP; absorbance maxima at 668 nm and 690 nm) |
| Amount used: | 30.15 mg |
| Test system: | Modified USP type II dissolution apparatus comprising two-layer water-octanol dissolution media plus a double paddle stirrer |
| Aqueous media: | 600 ml De-ionized water (pH 7) |
| Lipophilic media: | 400 ml Octanol |
| Temperature: | 37°C |
| Rotation Speed | 50 rpm |

The experiment in which the polar dye and the amphiphilic compound were both initially added to the aqueous layer resulted with time in the progressive development of blue color, and in an increase in the concentration of the polar agent in the octanol layer. In contrast, in the control experiment, there was no significant color development or increase in the concentration of the polar agent in the octanol layer. These results therefore indicate that in the absence of an amphiphilic compound, the polar agent is not significantly transported into the octanol layer. In the presence of an amphiphilic compound, however, reverse micelles can be formed with the polar agent, which can cross the water/octanol interface into the octanol phase.

### Transport of reverse micelles comprising polar basic agent and an anionic surfactant.

In this experiment, a metformin 500 mg tablet, prepared as described above, was dropped into an aqueous phase (de-ionised water or phosphate buffer) and allowed to dissolve. Thereafter, a layer of a octanol was slowly added to form a distinct layer above the aqueous layer. A double-paddle stirring device was used to ensure simultaneous agitation of both layers. Samples of 5ml were removed from the top (octanol) layer and analyzed spectrophotometrically at 232 nm for metformin content. The sampling times were taken at 30 min intervals post dissolution of the tablet in the aqueous layer. A similar experiment was conducted with the prior art formulation Glucophage XR (Metformin 500 mg). Figure 6 shows the results of the experiments. The specific details relating to the set-up of the experiment are shown below:

### Description of Apparatus:

| |
|---|
| **Vessel:** 4000 ml, 2000 ml, or 1000 ml Glass Beaker |
| **Agitator:** Double paddle rotating shaft |
| **Temperature:37** degree Celsius ± 0.5 degree Celsius |
| **Speed of Agitation:** 40 rpm, or 50 rpm |
| **Testing Media:** Aqueous phase: 900 ml or 600 ml of De-ionised water (pH7), |
| PBS (pH 6.8), or suitable aqueous media. Oil Phase: 400 ml or 200 ml Octanol or |
| suitable lipophilic media. The ratio of aqueous to oil phase can be experimentally |
| determined and may range from 1:0.25 to 1:1. |

The results shown in Figure 6 demonstrate that a polar agent, for example but not limited to metformin may be effectively transported across a lipophilic barrier by reverse micelle delivery system of the present invention.

### EXAMPLE 4: In-Vivo Drug Release

### Reverse-Micellar Metformin HCL Formulation

Metformin is an antihyperglycemic drug of the biguanide class used in the treatment of non-insulin dependent or type II diabetes mellitus (NIDDM). The immediate release dosage form and the extended release dosage forms are usually marketed in the form of its hydrochloride salt as Glucophage (TM-Bristol Myers Squibb) and Glucophage XR (TM-Bristol Myers Squibb) respectively.

Metformin hydrochloride is a class III biopharmaceutic drug.and has intrinsically poor permeability in the lower portion of the GIT leading to absorption almost exclusively in the upper part of the GIT.

Its oral bioavailability known in the art is in the range of 40 to 60%, and generally decreases with increasing dosage, which suggests a saturable absorption process, or permeability/transit time limited absorption. It also has a very high water solubility (> 300 mg/ml at 25 °C). This can lead to difficulty in providing a slow release rate from a formulation as well as achieving higher bioavailabilities from multiple doses. Metfomin is usually prescribed to be taken b.i.d. or t.i.d. or q.i.d. for type II diabetic patients who are unable to control their blood glucose with diet and exercise alone. The challenge with metformin is the lack of dose proportionality to the observed bioavailability when multiple doses are administered. This presents an impediment to the development of a once daily controlled release dosage form as a replacement for the conventional multiple doses. Traditionally a once or twice daily controlled release dosage form will contain an equivalent of multiple single doses in one dose to be released over a period of time, typically over a twelve or twenty-four hour time frame. In order for the sustained release dose to be effective, it should proffer a dose proportional or near-proportional bioavailability. Metformin and other similar class III biopharmaceutics drugs experience the aforementioned lack of dose-bioavailability relationship. The current invention has sought to overcome these problems by providing a bioavailability enhancing mechanism through reverse-micellar drug delivery. Such bioavailability enabling delivery system enhances the absorption of metfomin and other class III biopharmaceutics drug candidates.

Tablets containing 500 mg metformin hydrochloride prepared as described above or a prior-art Metformin 500 mg Extended release (Glucophage XR) tablets were dosed to 6 healthy male volunteers after an overnight fast. The study was a two-way crossover design with a one- week wash-out period between dosing arms. Blood samples were taken at 0.5, 1.0, 1.5, 2, 3, 5, 7, 9, 11, 13, 15, 17, 21, 24, and 30 hours post dose and analysed for metformin. The mean plasma concentration versus time plot and the area under the plasma concentration versus time (AUC) was calculated using the trapezoidal method. The AUC is indicative of the bioavailability of the drug. The plasma profiles and corresponding AUCs for two variations of the reverse-micellar Metformin formulations prepared as described herein (Example 1A) were compared with the prior-art Glucophage XR formulation in human subjects.

Figure 7 shows the results of comparative tests of the reverse micelle delivery system of the present invention, prepared according to the procedure of Example 1A (extended release form), and Glucophage XR.

Figure 8 shows the results of a reverse micelle delivery system as prepared according to Example 1B (delayed release form), and Glucophage XR. Example 1A is a controlled release formulation designed to start releasing its content in the gastric compartment. Example 1B is a delayed release formulation designed to release its content in the mid to lower gastro-intestinal tract.

The results suggest that the formulations may be employed to deliver metformin hydrochloride, achieve a higher bioavailability as well as enhance absorption in the mid to lower gastro-intestinal tract of a subject. Thus the delivery system of the present invention maybe employed in the treatment of NIDDM in human subjects.

Further aspects of the invention include the following:
A. A transmembrane delivery system comprising a reverse micelle and polar agent of interest.
B. The delivery system as defined in paragraph A, wherein said reverse micelle comprises at least one amphipathic ionic compound, and said polar agent of interest comprises at least one polar ionizable agent of interest.
C. The delivery system as defined in paragraph B, wherein said amphipathic compound is an anionic surfactant capable of forming micelles in a fluid environment.
D. The delivery system as defined in paragraph B, wherein said amphipathic compound is a cationic surfactant capable of forming micelles in a fluid environment.
E. The delivery system as defined in paragraph B, wherein said agent of interest is characterized by a partition coefficient between water and octanol at pH 7.4 of less than about 10.
F. The delivery system as defined in paragraph B, wherein said amphipathic compound is present in an amount of about 0.5 weight % to about 500 weight %.
G. The delivery system as defined in paragraph B, wherein said agent is a therapeutically active compound of a Class III biopharmaceutics classification and exhibits high solubility and low permeability.
H. The delivery system as defined in paragraph B, wherein the agent of interest comprises a plurality of discrete active particulates.
I. The delivery system as defined in paragraph B, wherein said amphipathic compound is an ionic surfactant or mixture of ionic surfactants selected from the group consisting of anionic surfactants, cationic surfactants and zwitterionic surfactants.
J. The delivery system as defined in paragraph I, wherein the anionic surfactants are selected from the group consisting of sodium or potassium dodecyl sulfate, sodium octadecylsulfate, sodium bis(2-ethylhexyl) sulfosuccinate (AOT), and a combination thereof
K. The delivery system as defined in paragraph I, wherein the cationic surfactants are selected from the group consisting of didodecyl dimethyl ammonium bromide (DDAB), cetyltriammonium bromide (CTAB), cetylpyridinium bromide (CPB), dodecyl trimethyl ammonium chloride (DOTAC), sodium perfluorononanoate (SPFN), hexadecyl trimethyl ammonium bromide (HDTMA), or a combination thereof
L. The delivery system as defined in paragraph I, formulated into a solid tablet, matrix tablet, granules or capsule.
M. The delivery system as defined in paragraph I, further comprising one or more pharmaceutically acceptable excipients.
N. The delivery system as defined in paragraph M, wherein said one or more pharmaceutically acceptable excipients is selected from the group consisting of one or more viscosity enhancers, enteric polymers, pH-specific barrier polymers, diluents, anti-adherents, glidants, binders, solubilizers, channeling agents, wetting agents, buffering agents, flavourants, adsorbents, sweetening agents, colorants, lubricants, and a combination thereof
O. The delivery system as defined in paragraph B, wherein the agent of interest is selected from the group consisting of one or more of an analgesic, anti-inflammatory, antimicrobial, amoebicidal, trichomonocidal agents, anti-Parkinson, anti-malarial, anticonvulsant, anti-depressants, antiarthritics, anti-fungal, antihypertensive, antipyretic, anti-parasite, antihistamine, alpha-adrenergic agonist, alpha blocker, anaesthetic, bronchial dilator, biocide, bactericide, bacteriostat, beta adrenergic blocker, calcium channel blocker, cardiovascular drug, contraceptive, decongestants, diuretic, depressant, diagnostic, electrolyte, hypnotic, hormone, hyperglycaemic, muscle relaxant, muscle contractant, ophthalmic, parasympathomimetic, psychic energizer, sedative, sympathomimetic, tranquilizer, urinary, vaginal, viricide, vitamin, non-steroidal anti-inflammatory, angiotensin converting enzyme inhibitors, polypeptide, proteins, sleep inducers, and a combination thereof
P. The delivery system as defined in paragraph I, wherein the system is derived from a matrix-type solid compact, made by a compression or pelletization method, or a matrix-type extrusion spheroid, made by a wet or dry extrusion method.
Q. The delivery system as defined in paragraph I, wherein said system is granulated or micro encapsulated to form particulates that may be compressed into solid compacts or filled into capsules.
R. The delivery system as defined in paragraph I, wherein said dosage form is selected from the group consisting of granulated, particulate, spheroidal, compact and dry blends, and wherein said system can be filled into capsules or suspended in a suitable liquid vehicle.
S. The use of the delivery system as defined in paragraph B, to deliver one or more therapeutic agents to a subject in need thereof.
T. A method of delivering a therapeutic agent to a subject in need thereof comprising,
   i) formulating the delivery system as defined in paragraph B such that the agent of interest comprises a therapeutic agent and;
   ii) administering said delivery system to a subject in need thereof
U. The method as defined in paragraph T, wherein said administering comprises oral administration.

All references are herein incorporated by reference.

The present invention has been described with regard to preferred embodiments. However, it will be obvious to persons skilled in the art that a number of variations and modifications can be made without departing from the scope of the invention as described herein.

## Claims

1. A pharmaceutical composition comprising a dry blended mixture comprising:
i) at least one surfactant wherein the at least one surfactant is an anionic surfactant or a cationic surfactant, and
ii) a polar ionizable therapeutic drug;
wherein the at least one surfactant and the polar ionizable therapeutic drug are oppositely charged at the pH of a single phase aqueous medium, and
wherein the dry blended mixture comprises a sufficient amount of the at least one surfactant such that, following dissolution of the dry blended mixture within the single phase aqueous medium, the at least one surfactant forms a reverse micelle comprising the polar ionizable therapeutic drug.

2. The composition according to claim 1, wherein the dry blended mixture comprises only one surfactant.

3. The composition according to claim 1 or 2, wherein the at least one surfactant is an anionic surfactant, and the polar ionizable therapeutic drug is positively charged.

4. The composition according to claim 1 or 2, wherein the at least one surfactant is a cationic surfactant, and the polar ionizable therapeutic drug is negatively charged.

5. The composition according to claim 3, wherein the anionic surfactant is selected from the group consisting of sodium or potassium dodecyl sulfate, sodium octadecylsulfate, sodium bis(2-ethylhexyl)sulfosuccinate (AOT), and a combination thereof.

6. The composition according to claim 4, wherein the cationic surfactant is selected from the group consisting of didodecyl dimethyl ammonium bromide (DDAB), cetyl-triammonium bromide (CTAB), cetylpyridinium bromide (CPB), dodecyl trimethyl ammonium chloride (DOTAC), sodium perfluorononanoate (SPFN), hexadecyl trimethyl ammonium bromide (HDTMA), and a combination thereof.

7. The composition according to any one of the preceding claims, wherein the polar ionisable drug has a partition coefficient between water and octanol at pH 7.4 of less than 10.

8. The composition according to any one of the preceding claims, wherein the polar ionisable drug is a compound of Class III of the Biopharmaceutics Classification System.

9. The composition according to any one of the preceding claims, wherein the polar ionizable therapeutic drug is selected from the group consisting of metformin, cimetidine, ranitidine, sodium cromoglycate, gabapentin, clodronate and captopril.

10. The composition according to any one of the preceding claims, wherein the dry blended mixture further comprises a pharmaceutically acceptable excipient selected from the group consisting of a diluent, a glidant, a binder, a buffering agent, a flavourant, an adsorbent, a sweetening agent, a colorant, a lubricant, and a combination thereof.

11. The composition according to any one of the preceding claims, wherein the polar ionizable therapeutic agent of interest is selected from the group consisting of an analgesic, an anti-inflammatory, an antimicrobial, an amoebicidal, a trichomonocidal agent, an anti-Parkinson, an anti-malarial, an anticonvulsant, an anti-depressant, an anti-arthritic, an anti-fungal, an antihypertensive, an antipyretic, an anti-parasite, an antihistamine, an alpha-adrenergic agonist, an alpha blocker, an anaesthetic, a bronchial dilator, a biocide, a bactericide, a bacteriostat, a beta adrenergic blocker, a calcium channel blocker, a cardiovascular drug, a contraceptive, a decongestant, a diuretic, a depressant, a diagnostic, an electrolyte, a hypnotic, a hormone, a hyperglycaemic, a muscle relaxant, a muscle contractant, an ophthalmic, a parasympathomimetic, a sedative, a sympathomimetic, a tranquilizer, a viricide, a vitamin, a non-steroidal anti-inflammatory, an angiotensin converting enzyme inhibitor, a polypeptide, a protein, a sleep inducer, and a combination thereof.

12. The composition according to any one of claims 1 to 9, wherein the composition further comprises an enteric polymer or a pH-specific barrier polymer.

13. The composition according to claim 1, wherein the at least one surfactant is present in an amount of 0.5 weight % to 500 weight %.

14. The pharmaceutical composition according to any one of the preceding claims wherein the pharmaceutical composition is formulated in a solid tablet, matrix tablet, granules or capsule.
